(19) Europäisches Patentamt
European Patent Office
Office européen des brevets

(11) Veröffentlichungsnummer : **0 030 922**
**B1**

(12) **EUROPÄISCHE PATENTSCHRIFT**

(45) Veröffentlichungstag der Patentschrift : **04.07.84**

(21) Anmeldenummer : **80810384.0**

(22) Anmeldetag : **12.12.80**

(51) Int. Cl.³ : **C 07 C127/19**, C 07 D213/61, C 07 D309/12, C 07 D295/20, A 01 N 47/32

(54) **Äthinyl-Phenylharnstoffe, Verfahren zu deren Herstellung und deren Verwendung als Herbizide.**

(30) Priorität : **18.12.79 CH 11214/79**

(43) Veröffentlichungstag der Anmeldung : **24.06.81 Patentblatt 81/25**

(45) Bekanntmachung des Hinweises auf die Patenterteilung : **04.07.84 Patentblatt 84/27**

(84) Benannte Vertragsstaaten : **BE CH DE FR GB IT LI NL**

(56) Entgegenhaltungen : **US-A- 2 655 447**

(73) Patentinhaber : **CIBA-GEIGY AG**
**Postfach**
**CH-4002 Basel (CH)**

(72) Erfinder : **Szczepanski, Henry, Dr.**
**Waldshuterstrasse 55**
**CH-4310 Rheinfelden (CH)**

**EP 0 030 922 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue Aethinyl-phenylharnstoffe mit herbizider Wirkung, Verfahren zu deren Herstellung, sie als Wirkstoffe enthaltende Mittel und deren Verwendung.

Die neuen Aethinyl-phenylharnstoffe entsprechen der Formel I

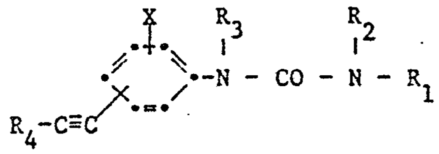

(I)

worin

X Wasserstoff, Halogen, $C_1$-$C_4$ Alkyl, $C_1$-$C_4$ Alkoxy, $C_1$-$C_4$ Haloalkyl oder Nitro,

$R_1$ Wasserstoff, $C_1$-$C_6$ Alkyl, $C_1$-$C_4$ Alkoxy, $C_2$-$C_6$ Alkenyl oder Phenyl, welches unsubstituiert oder durch Halogen substituiert sein kann,

$R_2$ Wasserstoff, $C_1$-$C_6$ Alkyl, $C_3$-$C_8$ Cycloalkyl, $C_2$-$C_6$ Alkenyl, $C_2$-$C_6$ Alkinyl oder Benzyl, welches unsubstituiert oder durch Halogen substituiert sein kann,

$R_1$ und $R_2$ zusammen mit dem Stickstoffatom an das sie gebunden sind, auch einen Pyrrolidin-, Piperidin-, Morpholin- oder Piperazinring bilden können,

$R_3$ Wasserstoff oder $C_1$-$C_4$ Alkyl,

$R_4$ Wasserstoff, $C_1$-$C_{12}$ Alkyl, welches unsubstituiert oder durch Hydroxy, Halogen, Pyranyloxy, Furanyloxy, Phenyl, Phenoxy, $C_1$-$C_4$-Alkoxy, $C_2$-$C_8$ Alkoxyalkoxy, $C_1$-$C_4$ Alkoxycarbonyl oder eine Aminogruppe —$N(R_1)R_2$, —$N(R_1)COOR_2$, —$N(R_1)COSR_2$ oder durch —$CON(R_1)R_2$ substituiert ist,

— Phenyl oder Pyridyl, welches unsubstituiert oder durch Halogen, $C_1$-$C_4$ Alkyl, $C_1$-$C_4$ Alkoxy, $C_1$-$C_4$ Haloalkyl, Nitro, $C_1$-$C_4$ Alkylsulfonyl oder Phenoxy substituiert ist, wobei Phenoxy unsubstituiert oder wie Phenyl substituiert ist,

— $C_2$-$C_{12}$ Alkenyl, welches unsubstituiert oder durch Halogen, Hydroxy, $C_1$-$C_4$ Alkoxy, $C_2$-$C_8$ Alkoxyalkoxy oder $C_1$-$C_4$ Alkoxycarbonyl substituiert ist,

— $C_3$-$C_{12}$ Cycloalkyl oder Cycloalkenyl, welches mono- oder bicyclisch ist und durch Halogen, Hydroxy oder $C_1$-$C_4$ Alkoxy substituiert sein kann.

Die Alkylgruppe $R_4$ kann unverzweigt sein, ist aber vorteilhafterweise ein in der 1-Stellung verzweigter Rest, der auch noch substituiert sein kann wie beispielsweise Isopropyl, 1-Hydroxyisopropyl, 1-Chlorisopropyl, 2-Butyl, 2-Hydroxylbutylreste etc.

Die Alkenylreste $R_4$ sind ebenfalls bevorzugt in der 1-Stellung substituiert, wie But-3-en-2-yl, Prop-2-en-2-yl. Die Alkyl- und Alkenylreste können durch Halogen oder Hydroxyl substituiert und/oder durch Sauerstoffbrücken ein- oder mehrfach unterbrochen sein. Zu den Substituenten dieser Gruppen gehören auch die Pyranyloxy- und der Furanyloxyrest oder ein Pyridyloxyrest. Bevorzugt als Cycloalkyl- oder Cycloalkenylreste sind Cyclopentyl, Cyclohexyl und Cyclohex-1-enyl. Weitere bevorzugte Reste $R_4$ sind über ein oder zwei Sauerstoffatome verbundene bis tertiäre Alkyl- und Alkenylgruppen wie beispielsweise die 2-(1'-Aethoxy-äthoxy)-but-2-yl-gruppe, die 3-(1'-Aethoxy-äthoxy)-pent-3-ylgruppe etc.

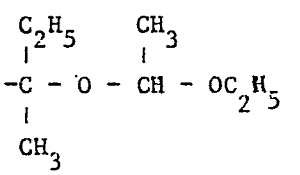

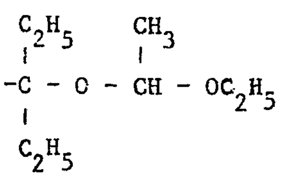

In der US-Patentschrift No. 2 655 447 und in der schweizerischen Patentschrift CH-A-583 509 werden Alkenylphenylharnstoffe mit herbizider Wirkung erwähnt, welche ohne praktische Bedeutung geblieben sind wegen ihrer im Vergleich zu anderen Phenylharnstoffen schwächeren Wirkung.

Die erfindungsgemässen Aethinyl-harnstoffe der Formel I beeinflussen das Pflanzenwachstum und zeigen insbesondere ausgezeichnete herbizide Eigenschaften. Sie dienen vor allem der Unkrautbekämpfung, können aber auch aufgrund ihrer vorteilhaften austrocknenden und entblätternden Wirksamkeit als Erntehilfsmittel in Kulturen wie Baumwolle oder Kartoffeln eingesetzt werden.

Bei der Bekämpfung mono- und dikotyler Unkräuter zeigen die Wirkstoffe der Formel I selbst bei geringen Aufwandmengen eine hervorragende herbizide Wirkung auf unerwünschten Pflanzenwuchs unter deutlicher Schonung einer Reihe von Nutzpflanzenkulturen und sind strukturell ähnlich bekannten Phenylharnstoff-Derivaten überraschend überlegen. Es werden dabei auch schwer bekämpfbare Unkrautarten erfasst.

Bei genügend grosser Aufwandmenge ist jedoch auch totalherbizide Wirkung vorhanden. Die Anwendung kann sowohl im Vorauflauf- wie im Nachauflaufverfahren vorgenommen werden. Dabei können die Aufwandmengen in weiten Grenzen schwanken, z. B. zwischen 0,1 bis 10 kg Wirkstoff pro

Hektare, vorzugsweise werden jedoch 0,5 bis 5 kg Wirkstoff pro Hektare eingesetzt.

Die erfindungsgemässen Mittel enthalten ausser dem Wirkstoff der Formel I einen geeigneten Träger und/oder andere Zuschlagstoffe. Geeignete Träger- und Zuschlagstoffe können fest oder flüssig sein und entsprechen den in der Formulierungstechnik üblichen Stoffen wie z. B. natürlichen oder regenerierten mineralischen Stoffen, Lösungs-, Verdünnungs-, Dispergier-, Emulgier-, Netz-, Haft-, Verdickungs-, Binde- und/oder Düngemitteln.

Zur Verwendung in herbiziden Mitteln kann die Verbindung der Formel I als Stäubemittel, Emulsionskonzentrat, als Granulat, Dispersion oder auch als Lösung oder Aufschlämmung in üblicher Formulierung verarbeitet werden.

Als besonders aktiv haben sich folgende Untergruppen von Aethinyl-phenylharnstoffen erwiesen, welche alle von der Formel I umfasst werden :

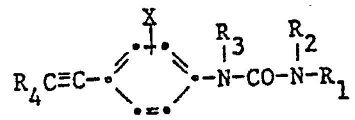

(Ia)

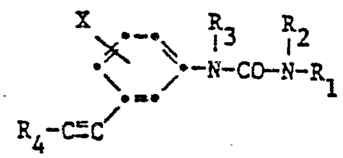

(Ib)

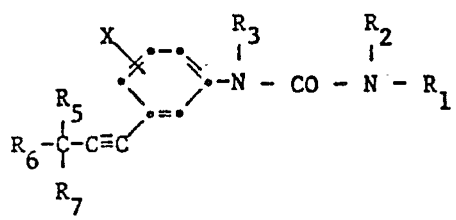

(Ic)

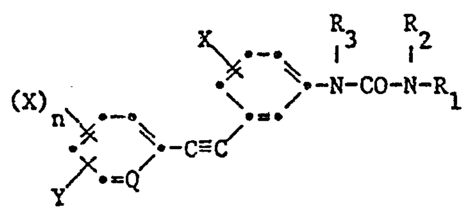

(Id)

In diesen Formeln haben $R_1$, $R_2$, $R_3$, $R_4$ und X die unter Formel I gegebene Bedeutung, während

$R_5$ Wasserstoff, $C_1$-$C_6$ Alkyl, $C_2$-$C_6$ Alkenyl,

$R_6$ dasselbe wie $R_5$ oder Phenyl der unsubstituiert oder durch Halogen, $C_1$-$C_4$ Alkyl, $C_1$-$C_4$ Alkoxy, $C_1$-$C_4$ Haloalkyl oder Nitro substituiert ist, $R_5$ und $R_6$ zusammen mit dem Kohlenstoffatom, an das sie gebunden sind auch einen $C_3$-$C_{12}$ Cycloalkyl- oder Cycloalkenylrest darstellen kann, der mono- oder bicyclisch ist und der durch Halogen, Hydroxy oder $C_1$-$C_4$ Alkoxy substituiert sein kann,

$R_7$ Hydroxy, $C_1$-$C_6$ Alkyl, welches durch Halogen oder Hydroxy substituiert und durch Sauerstoff ein- oder mehrfach unterbrochen sein kann, wobei die Anzahl der Kohlenstoffatome in den Resten $R_5$, $R_6$ und $R_7$ 11 nicht übersteigt,

Q eine Methingruppe oder ein Stickstoffatom,

Y Wasserstoff, Halogen, Nitro oder einen gegebenenfalls durch Halogen, Nitro, $C_1$-$C_4$ Alkyl, $C_1$-$C_4$ Alkoxy oder $C_1$-$C_4$ Haloalkyl ein- oder mehrfach substituierten Phenoxyrest,

n 0 oder 1 bedeuten.

Die Herstellung der Verbindungen der Formel I in denen $R_3$ Wasserstoff bedeutet erfolgt nach an sich bekannten Methoden, z. B. durch Reaktion eines Aethinyl-phenylderivates der Formel II

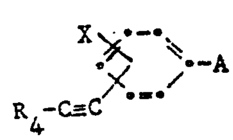

(II)

mit einem Amin der Formel III

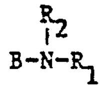

(III)

worin $R_1$, $R_2$, $R_4$ und X die unter Formel I gegebene Bedeutung haben, während A und B Reste bedeuten, die unter Anlagerung oder Kondensation Harnstoffe bilden. Dabei stellt eine der beiden Gruppen A und B ein Amin dar, während die andere ein Urethan, ein Carbamoyhalogenid, eine Harnstoffgruppe, oder insbesondere den Isocyanatrest darstellt.

Ein weiterer Weg zur Herstellung der Aethinyl-phenylharnstoffe der Formel I besteht darin, dass man einen halogenierten Phenylharnstoff der Formel IV,

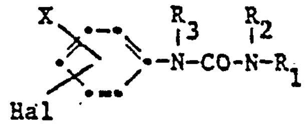

(IV)

worin $R_1$, $R_2$, $R_3$ und X die oben gegebene Bedeutung haben und Hal ein sich in der meta- oder para-Position zur Harnstoffgruppe befindendes Halogenatom, insbesondere ein Chlor- oder Brom- oder Jodatom bedeutet, in Gegenwart einer Base mit einer Aethinylverbindung der Formel V

$$R_4—C{\equiv}CH \qquad (V)$$

umsetzt, wobei $R_4$ die oben gegebene Bedeutung hat.

In dieser Umsetzung kann anstatt einer Aethinylverbindung der Formel V in Gegenwart einer starken, anorganischen Base wie NaOH oder KOH auch ein tertiärer Aethinylalkanol der Formel VI verwendet werden

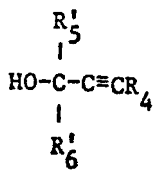

(VI)

worin $R'_5$ und $R'_6$ die oben für $R_5$ und $R_6$ gegebene Bedeutung haben, mit Ausnahme von Wasserstoff. Bei dieser Umsetzung wird ein Ketorest $O = CR'_5R'_6$ abgespalten.

In gleicher Weise gelingt es, einen tertiären Alkanolrest $R_4$ vom Aethinylrest abzuspalten und das verbleibende Wasserstoffatom in Gegenwart einer Base mit einem anderen Rest, beispielsweise einem halogenierten Rest Hal $R_4$, beispielsweise einem Phenylrest umzusetzen entsprechend dem Schema

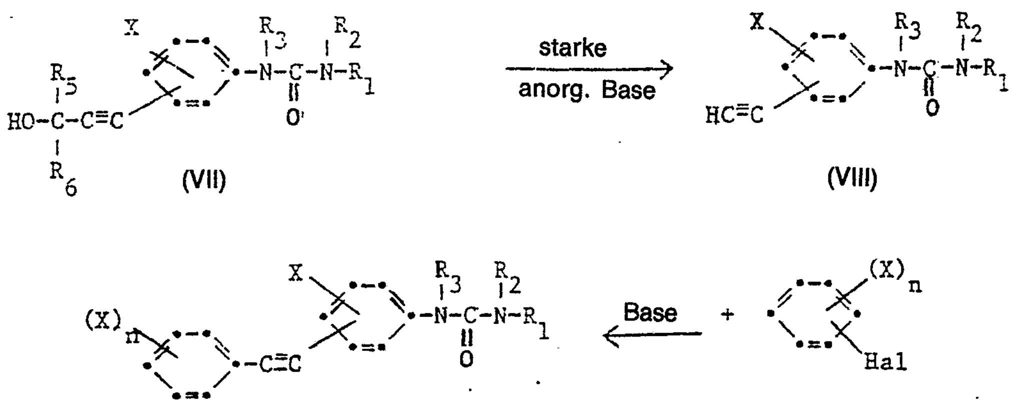


(VII) (VIII)

In diesen Formeln haben $R_1$, $R_2$, $R_3$, $R_4$, $R_5$, $R_6$, X, Hal und n die oben gegebene Bedeutung.

Diese Umsetzungen finden vorteilhafterweise in den Reaktionspartnern gegenüber inerten, organischen Lösungsmitteln oder Lösungsmittelgemischen statt. Als solche kommen viele protische wie auch aprotische in Frage, z. B. Alkanole, Ketone, Aether, Kohlenwasserstoffe, halogenierte Kohlenwasserstoffe, aromatische Lösungsmittel oder auch z. B. Dimethylformamid oder Dimethylsulfoxid.

Als säurebindendes Mittel kann, wenn bei der Umsetzung ein Halogenatom abgespalten wird, eine Base eingesetzt werden. Dafür kommen starke anorganische Basen wie KOH, NaOH in Frage, aber auch

organische wie beispielsweise Trimethylamin, Diäthylamin, Pyridin etc.

Die Umsetzungen in denen die Aethinylgruppe miteinbezogen ist, werden mit Vorteil in Gegenwart eines Katalysators durchgeführt. Als solche kommen vor allem Edelmetallkatalysatoren in Frage, welche als solche oder auf einen Träger wie z. B. Kohlepulver, Aluminiumoxyd etc. aufgezogen sind, eingesetzt werden. Es kommen Palladiumkomplexe in Frage welche man eventuell unter Zusatz von Kupferjodid (CuI) verwendet. Beispiele solcher Katalysatoren sind Palladiumacetat $Pd(OCOCH_3)_2$ der Palladium-dichlor-bis-(triphenylphosphin)-Komplex $PdCl_2[P(C_6H_5)_3]_2$.

Die Temperaturen der Umsetzungen liegen zwischen Raumtemperatur und dem Siedepunkt des Reaktionsgemisches. Während Umsetzungen, die zur Synthese der Harnstoffgruppe führen, normalerweise schon bei Raumtemperatur erfolgen und auch die diejenigen Umsetzungen in denen die Aethinylgruppe miteinbezogen oft leicht exotherm verlaufen, wird zur Steigerung der Reaktionsgeschwindigkeit gerne während kurzer Zeit die Temperatur im Reaktionsgefäss erhöht.

Ausgangsprodukte der Formel II werden z. B. in der DOS 2 905 507 und im US-Patent No. 4 128 588 beschrieben. Um eine Verbindung der Formel II herzustellen, worin A = $NH_2$ bedeutet, d. h. ein Anilin, reduziert man beispielsweise ein acetylensubstituiertes Nitrobenzol. Die Nitrogruppe lässt sich selektiv hydrieren, welches mit speziellen Reduktionsmitteln, wie Zink, Zinksalze, Eisensalze, Natrium- und Ammonium-sulfit, -dithionit oder -hydrosulfit geht, aber auch durch katalytische Hydrierung mit speziellen Katalysatoren auf der Basis von Ruthenium oder Schwermetalloxiden.

Die Ausgangsmaterialien der Formeln III, IV, V und VI sind bekannt oder leicht herstellbar und/oder kommerziell erhältlich.

Die Verbindungen der Formel I sind in den üblichen organischen Lösungsmitteln relativ gut und in Wasser schlecht löslich.

Ihre Formulierung als flüssige herbizide Mittel gelingt mit Hilfe besonderer Lösungsvermittler und/oder Dispergiermittel.

Die neuen Wirkstoffe der Formel I sind stabile Verbindungen, welche in üblichen organischen Lösungsmitteln, wie Alkoholen, Aethern, Ketonen, Dimethylformamid, Dimethylsulfoxyd etc. löslich sind.

Die Herstellung erfindungsgemässer Mittel erfolgt in an sich bekannter Weise durch inniges Vermischen und Vermahlen von Wirkstoffen der allgemeinen Formel I mit geeigneten Trägerstoffen und/oder Verteilungsmitteln, gegebenenfalls unter Zusatz von gegenüber den Wirkstoffen inerten Antischaum-, Netz-, Dispersions- und/oder Lösungsmitteln. Die Wirkstoffe können in den folgenden Aufarbeitungsformen vorliegen und angewendet werden :

Feste Aufarbeitungsformen : Stäubemittel, Streumittel, Granulate, Umhüllungsgranulate, Imprägnierungsgranulate und Homogengranulate ;

In Wasser dispergierbare Wirkstoffkonzentrate : Spritzpulver (wettable powder), Pasten, Emulsionen ; Emulsionskonzentrate Flüssige Aufarbeitungsformen : Lösungen.

Die Wirkstoffkonzentrationen betragen in den erfindungsgemässen Mitteln 1 bis 80 Gewichtsprozent und können gegebenenfalls bei der Anwendung in geringen Konzentrationen wie etwa 0,05 bis 1 % vorliegen.

Den beschriebenen erfindungsgemässen Mitteln lassen sich andere biozide Wirkstoffe oder Mittel beimischen. So können die neuen Mittel ausser den genannten Verbindungen der allgemeinen Formel I z. B. Insektizide, Fungizide, Bakterizide, Fungistatika, Bakeriostatika, Nematozide oder weitere Herbizide zur Verbreiterung des Wirkungsspektrum enthalten.

Die folgenden Beispiele sollen die Herstellung der erfindungsgemässen Phenylharnstoffe der Formel I näher erläutern. Weitere in analoger Weise hergestellte Verbindungen sind in der anschliessenden Tabelle aufgeführt. Die Temperaturen sind in Celsiusgraden angegeben, Teile und Prozentangaben beziehen sich auf das Gewicht, Druckangaben werden in Millibar (mbar) angegeben.

## Beispiel 1

N-[3-Chlor-4-(3'-methyl-3'-hydroxybut-1'-in-1'-yl)-phenyl]-N'-methoxy-N'-methyl-harnstoff

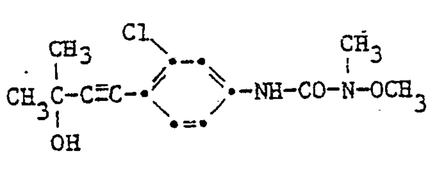

Zu einer Lösung von 69,4 g (0,25 Mol) N-(3'-Chlor-4'-bromphenyl)-N'-methoxy-N'-methylharnstoff und 30 g (0,36 Mol) 3-Hydroxy-3-methyl-1-butin in einem Gemisch von 500 ml Triäthylamin und 15 ml Dimethylformamid wurden unter Stickstoff 0,4 g Kupferjodid und 1,6 g Palladiumkomplex $PdCl_2[P(C_6H_5)_3]_2$ gegeben.

Das Reaktionsgemisch wurde 24 Stunden auf 80 °C erwärmt, dann mit etwas Aktivkohle versetzt, abgenutscht und eingedampft. Der Rückstand wurde in 500 ml Aether aufgenommen und dreimal mit je 300 ml 5 %iger wässriger HCl-Lösung gewaschen. Anschliessend wurde getrocknet, mit etwas Aktivkohle behandelt, abgenutscht und eingedampft.

Nach dem Eindampfen wurden 200 ml Aether zugegeben und das Produkt kristallisieren gelassen. Es wurden 44 g farbloser Kristalle der Titelverbindung vom Schmelzpunkt 123-124 °C erhalten.

## Beispiel 2

N-[3-Chlor-4-(3'-methyl-but-3'-en-1'-in-1'-yl)-phenyl]-N'-methoxy-N'-methyl-harnstoff

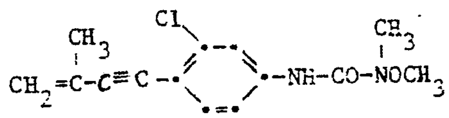

Eine Lösung von 15 g des Harnstoffes vom Beispiel 1 in 100 ml Toluol wurde in 10 ml Pyridin und 5,7 g Methylsulfonylchlorid $CH_3SO_2Cl$ bei Raumtemperatur versetzt. Dann wurde 4 Stunden auf 90 °C erwärmt. Anschliessend mit 300 ml Aether verdünnt und zweimal mit je 250 ml 5 %iger HCl-Lösung und 250 ml 5 %iger NaOH-Lösung gewaschen.

Die organische Phase wurde mit Aktivkohle versetzt, getrocknet, filtriert und eingedampft.

Das Rohprodukt wurde an Kieselgel chromatographiert (Laufmittel Methylenchlorid), wobei 5,2 g der Titelverbindung mit Schmelzpunkt 124-125 °C aus dem resultierenden Oel auskristallisierten.

## Beispiel 3

N-[3-(3'-Hydroxy-3'-methyl-but-1'-in-1'-yl)-phenyl]-N'-methoxy-N'-methyl-harnstoff

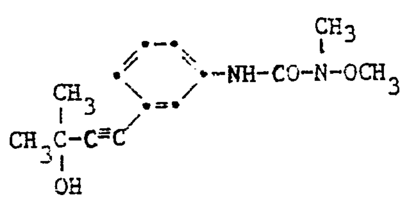

45 g N-(3-Jodphenyl)-N'-methoxy-N'-methyl-harnstoff wurden in 300 ml Triäthylamin suspendiert und unter Stickstoffatmosphäre mit 0,2 g Kupferjodid sowie 0,8 g Palladiumkomplex $PdCl_2[P(C_6H_5)_3]_2$ versetzt. Das Reaktionsgemisch wurde 14 Stunden bei 45 °C gerührt. Dann wurde mit Aether verdünnt und die Aetherlösung wie im Beispiel 1 angegeben sauer gewaschen, getrocknet und eingedampft. Der Rückstand wurde aus Hexan kristallisiert und ergab 30 g der Titelverbindung vom Smp. 76-78 °C.

## Beispiel 4

N-[3-(3'-Methyl-but-3'-en-1'-in-1'yl)-phenyl]-N'-methoxy-N'-methyl-harnstoff

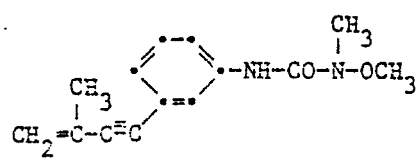

10,5 g des Harnstoffes vom Beispiel 3 wurden analog zum Verfahren vom Beispiel 2 mit Pyridin und Methylsulfonylchlorid versetzt und 4 Stunden auf 90 °C erwärmt. Nach dem Abkühlen wird das Reaktionsgemisches zur Trockne verdampft und der Rückstand in Aether aufgenommen. Nach dem Aufarbeiten wurde die organische Phase eingeengt und mittels Methylenchlorid über eine Kieselgel-Kolonne chromatographiert. Man erhielt so 6,1 g der Titelverbindung als hellbraunes Pulver vom Schmelzpunkt 56-67 °C.

## Beispiel 5

N-[3-(4'-Methyl-phenyl-äthinyl)-phenyl]-N'-methoxy-N'-methyl-harnstoff

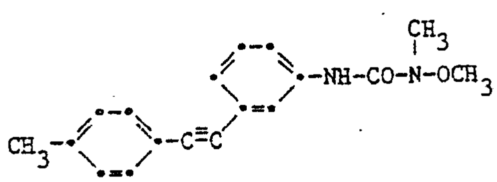

Eine Lösung von 11 g 3-(4'-Methyl-phenyl-äthinyl)-anilin in 100 ml Aether wurden mit 9 ml Triäthylamin und 6 g Methoxymethylcarbamoylchlorid versetzt und 4 Stunden am Rückfluss gekocht. Anschliessend wurde mit je 150 ml 5 %iger wässriger HCl-Lösung und Wasser gewaschen, getrocknet und die organische Phase eingedampft. Der Rückstand wurde in etwas Aether aufgenommen und kristallisiert. Man erhielt 12,5 g der Titelverbindung mit einem Schmelzpunkt von 113 °C.

Das als Zwischenprodukt benötigte Anilin wurde wie folgt hergestellt :

a) Zu einer Lösung von 44 g 3-Jodanilin und 20 g 3-Hydroxy-3-methyl-but-1-in in 200 ml Triäthylamin wurden unter Stickstoffatmosphäre mit 0,3 g Kupferjodid und 0,7 g Palladiumkomplex $PdCl_2[P(C_6H_5)_3]_2$ gegeben. Eine leicht exotherme Reaktion setzt ein, wobei die Temperatur bis 55 °C steigt. Anschliessend wird 12 Stunden bei 40 °C gerührt und das Reaktionsgemisch mit Aktivkohle versetzt, filtriert und eingedampft. Der Rückstand wurde in Aether aufgenommen und mit 5 %iger HCl-Lösung und Wasser gewaschen, getrocknet und eingedampft. Der Rückstand wurde mit etwas Aether versetzt und kristallisierte. Man erhielt so 30 g 3-(3'-Hydroxy-3'-methyl-but-1'-in-1'-yl)-anilin vom Schmelzpunkt 117-119 °C.

b) 17,5 g des obigen Anilins wurden in 100 ml Triäthylamin gelöst und mit 24 g 4-Jodtoluol versetzt. Dann wurden unter Stickstoffatmosphäre 0,2 g Kupferjodid und 0,7 g Palladiumkomplex $PdCl_2[P(C_6H_5)_3]_2$ sowie 10 g pulverisiertes KOH zugegeben und das Gemisch 14 Stunden bei 65 °C gerührt. Anschliessend wurde das Reaktionsgemisch mit Aktivkohle versetzt, abgenutscht und eingedampft. Der Rückstand wurde in 300 ml Aether gelöst und die Aetherlösung dreimal mit je 250 ml 10 %iger Kaliumkarbonatlösung gewaschen. Die organische Phase wurde mit Aktivkohle versetzt, filtriert, getrocknet und eingedampft. Der Rückstand wurde aus Hexan kristallisiert und lieferte 16 g 3-(4'-Methylphenyl-äthinyl)-anilin vom Schmelzpunkt 88 °C.

## Beispiel 6

N-{3-[4'-(2''-Chlor-4''-trifluormethylphenoxy-)phenyläthinyl-]phenyl}-N'-methoxy-N'-methyl-harnstoff

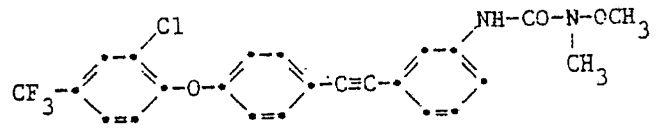

8 g 4-(2'-Chlor-4'-trifluormethylphenoxy)-1-äthinylbenzol und 8,25 g N-(3-Jodphenyl)-N'-methoxy-N'-methylharnstoff wurden in 70 ml Triäthylamin suspendiert und unter Stickstoffatmosphäre mit 0,1 g Kupferjodid und 0,2 g Palladiumkomplex $PdCl_2[P(C_6H_5)_3]_2$ versetzt. Es entstand eine leicht exotherme Reaktion, die Temperatur stieg auf 52 °C. Nachdem man die Reaktionsmishung noch 2 Stunden bei Raumtemperatur gerührt hatte, wurde etwas Aktivkohle zugegeben und abgenutscht. Das Filtrat wurde eingedampft und der Rückstand in Aether aufgenommen und dreimal mit 5 %iger wässriger Salzsäure gewaschen. Die organische Phase wurde getrocknet, mit Aktivkohle behandelt, filtriert und zur Trockne eingedampft. Der Rückstand wurde aus Aether/Hexan 1 : 5 auskristallisiert und man erhielt 8,1 g der Titelverbindung mit Schmelzpunkt 118-121 °C.

Analog zu diesen Beispielen wurden folgende Verbindungen hergestellt :

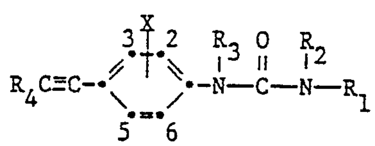

| Vbg.No. | X | $R_4$ | $R_3$ | $-NR_1R_2$ | phys. Daten |
|---|---|---|---|---|---|
| 1.1 | - | $C(CH_3)_2OH$ | H | $N(CH_3)OCH_3$ | Smp. 128-30° |
| 1.2 | - | $C(CH_3)_2OCONH$ 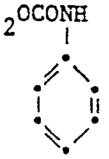 | H | $N(CH_3)OCH_3$ | Smp. 128°(Z) |
| 1.3 | - | $C(CH_3)=CH_2$ | H | $N(CH_3)OCH_3$ | Smp. 126° |
| 1.4 | 3-Cl | $C(CH_3)_2OH$ | H | $N(CH_3)OCH_3$ | Smp. 123-124° |
| 1.5 | 3-Cl | $C(CH_3)=CH_2$ | H | $N(CH_3)OCH_3$ | Smp. 124-125° |

(Fortsetzung)

| Vbg. No | X | $R_4$ | $R_3$ | $NR_1R_2$ | phys. Daten |
|---|---|---|---|---|---|
| 1.6 | 3-Cl | $CH(CH_3)OH$ | H | $N(CH_3)OCH_3$ | Smp. 90-92° |
| 1.7 | 3-Cl | $CH(CH_3)Cl$ | H | $N(CH_3)OCH_3$ | Smp. 103° |
| 1.8 | - | $C(CH_3)_2OH$ | H | $N(CH_3)_2$ | Smp. 115° |
| 1.9 | - | $C(CH_3)=CH_2$ | H | $N(CH_3)_2$ | |
| 1.10 | 3-Cl | $C(CH_3)OH$ | H | $N(CH_3)_2$ | |
| 1.11 | - | $C(CH_3)_2OCH_3$ | H | $N(CH_3)OCH_3$ | |
| 1.12 | - | $C(CH_3)=CH_2$ | H | $N(CH_3)OCH_3$ | Oel |
| 1.13 | - | $C(CH_3)(C_3H_7)OH$ | H | $N(CH_3)OCH_3$ | Oel |
| 1.14 | - | CH(OH)- 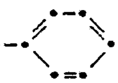 | H | $N(CH_3)OCH_3$ | Oel |
| 1.15 | - | $C(CH_3)_3$ | H | $N(CH_3)OCH_3$ | |
| 1.16 | - | $CH_3$ | H | $N(CH_3)OCH_3$ | |
| 1.17 | - | 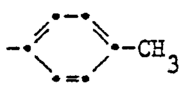 | H | $N(CH_3)OCH_3$ | |
| 1.18 | - | 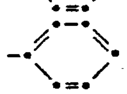 | H | $N(CH_3)OCH_3$ | |
| 1.19 | 3-Cl | $C(CH_3)_2OCH(CH_3)OC_2H_5$ | H | $N(CH_3)OCH_3$ | Oel |
| 1.20 | - | $C(CH_3)(C_3H_7)OCH(CH_3)OC_2H_7$ | H | $N(CH_3=OCH_3$ | Oel |
| 1.21 | - | $C(CH_3)=CH_2$ | H | $N(C_2H_5)_2$ | |
| 1.22 | - | $C(CH_3)_2OH$ | H | $N(C_2H_5)_2$ | |
| 1.23 | 3-Cl | H | H | $N(CH_3)OCH_3$ | Smp.103-4° |
| 1.24 | - | H | H | $N(CH_3)OCH_3$ | |

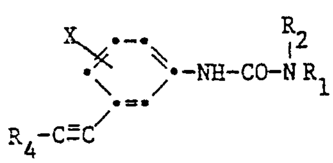

| Vbg.No. | X | $R_4$ | $NR_1R_2$ | phys. Daten |
|---|---|---|---|---|
| 2.1 | - | $-C(CH_3)(C_2H_5)OH$ | $N(CH_3)(OCH_3)$ | Smp. 79-81° |
| 2.2 | - | $-C(CH_3)(C_2H_5)OCH(CH_3)OC_2H_5$ | $N(CH_3)_2$ | $n_D^{21}$: 1.5210 |
| 2.3 | - | $-C(CH_3)=CH-CH_3$ | $N(CH_3)OCH_3$ | Oel |

(Fortsetzung)

| Vbg.No. | X | $R_4$ | $NR_1R_2$ | phys. Daten |
|---|---|---|---|---|
| 2.4 | - | $-C(CH_3)(C_2H_5)Cl$ | $N(CH_3)OCH_3$ | |
| 2.5 | - | $-C(C_2H_5)_2OH$ | $N(C_2H_5)_2$ | |
| 2.6 | - | $-C(C_2H_5)_2OCH(CH_3)OC_2H_5$ | $N(CH_3)_2$ | |
| 2.7 | - | $-C(C_2H_5)_2O-$ 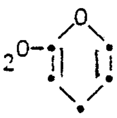 | $N(CH_3)OCH_3$ | |
| 2.8 | - | $-C(C_2H_5)=CH-CH_3$ | $N(CH_3)_2$ | Oel |
| 2.9 | - | $-C(C_2H_5)_2Cl$ | $N(CH_3)OCH_3$ | |
| 2.10 | - | $-C(CH_3)=CH-C_2H_5$ | $N(CH_3)OCH_3$ | |
| 2.11 | - | $-C(CH_3)(C_3H_7n)Cl$ | $N(C_2H_5)_2$ | |
| 2.12 | - | $-C(CH_3)(C_4H_9iso)OH$ | $N(CH_3)OCH_3$ | Oel |
| 2.13 | - | $-C(CH_3)(C_4H_9n)OCH(CH_3)OC_2H_5$ | $N(CH_3)_2$ | Oel |
| 2.14 | - | $-C(CH_3(C_4H_9n)O-$ 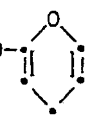 | $N(CH_3)OCH_3$ | |
| 2.15 | - | $-C(CH_3)(C_4H_9iso)Cl$ | $N(CH_3)OCH_3$ | |
| 2.16 | - | $-C(CH_3)=CH-C_3H_7iso$ | $N(CH_3)_2$ | Oel |
| 2.17 | - | $-C_4H_9n$ | $NHC_3H_7iso$ | |
| 2.18 | - | $-CH=CH-CH_2-OH$ | $NHC_4H_9n$ | |
| 2.19 | - | $-CH_2OH$ | $N(C_2H_5)_2$ | |
| 2.20 | - | $-CH=CH-CH_2OH$ | $N(CH_3)_2$ | |
| 2.21 | - | $CH=CH-CH_2OCH(CH_3)OC_2H_5$ | $N(CH_3)_2$ | |
| 2.22 | - | 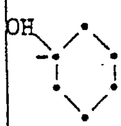 | $N(CH_3)OCH_3$ | Smp. 91-92° |
| 2.23 | | 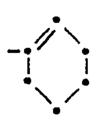 | $N(CH_3)OCH_3$ | Smp. 56-62° |
| 2.24 | - | $OCH(CH_3)OC_2H_5$ 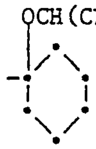 | $N(CH_3)OCH_3$ | $n_D^{21}$: 1.5295 |

(Fortsetzung)

| Vbg.No. | X | $R_4$ | $NR_1R_2$ | phys. Daten |
|---|---|---|---|---|
| 2.25 | - | 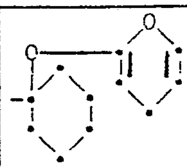 | $N(CH_3)_2$ | |
| 2.26 | - | $CH=CH_2$ | $N(CH_3)OCH_3$ | |
| 2.27 | - | $CH(CH_3)O-$ 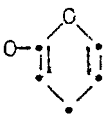 | $N(C_2H_5)_2$ | |
| 2.28 | - | $C(CH_3)(CH=CH_2)OH$ * | $N(CH_3)_2$ | |
| 2.29 | - | $C(CH_3)(CH=CH_2)Cl$ * | $N(CH_3)_2$ | *cis- und trans Form |
| 2.30 | - | $C(CH_3)(CH=CH_2)O-$ 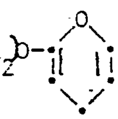 * | $N(CH_3)_2$ | |
| 2.31 | - | $C(CH_3)=CH-CH_2OCH(CH_3)$ * <br> $OC_2H_5$ | $N(CH_3)_2$ | |
| 2.32 | - | $C(CH=CH_2)=CH_2$ | $N(CH_3)OCH_3$ | |
| 2.33 | - | 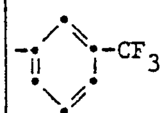 | $N(CH_3)_2$ | |
| 2.34 | - | 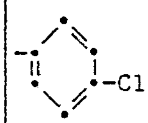 | $N(CH_3)C_4H_9n$ | |
| 2.35 | | 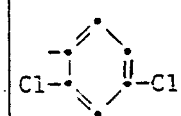 | $N(OCH_3)CH_3$ | Smp. 92-95° |
| 2.36 | | 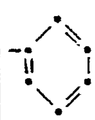 | $N(CH_3)_2$ | Smp. 101° |
| 2.37 | - | 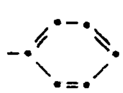 | $N(CH_3)(OCH_3)$ | Smp. 101° |

(Fortsetzung)

| Vbg.No. | X | $R_4$ | $NR_1R_2$ | phys. Daten |
|---|---|---|---|---|
| 2.38 | – | 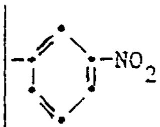 | $N(CH_3)OCH_3$ | |
| 2.39 | – | $CH(CH_3)_2OH$ | $N(CH_3)CH_2-CH=CH_2$ | |
| 2.40 | – | $CH(CH_3)=CHCH_3$ | $NHCH(CH_3)-CH=CH_2$ | |
| 2.41 | – | $C(CH_3)_3$ | $N(CH_3)_2$ | |
| 2.42 | – | $CH_3$ | $N(CH_3)OCH_3$ | Smp.85-86° |
| 2.43 | – | $C_4H_9n$ | $N(CH_2-CH=CH_2)_2$ | |
| 2.44 | – | $CH_2OH$ | $NHC_4H_9n$ | |
| 2.45 | – | $CH=CH_2$ | $NHC_3H_7iso$ | |
| 2.46 | – | $C(CH_3)_2O-$ 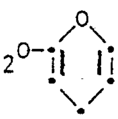 | $NH(CH_3)_2$ | |
| 2.47 | – | $CH_2O-$ 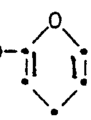 | $N(CH_3)_2$ | |
| 2.48 | – | H | $N(CH_3)OCH_3$ | Smp. 77-78° |
| 2.49 | – | $C(CH_3)(CH=CH_2)OH$ | $N(CH_3)OCH_3$ | Smp. 64-66° |
| 2.50 | – | $C(CH_3)(C_4H_9iso)OCH(CH_3)C_2H_5$ | $N(CH_3)OCH_3$ | Oel |
| 2.51 | – | $C(CH_3)=CH-C_3H_7iso$ | $N(CH_3)OCH_3$ | Oel |
| 2.52 | – | $CH_2OH$ | $N(CH_3)OCH_3$ | Oel |
| 2.53 | – | $C(CH_3)=CH\ CH_2OH$ (cis) | $N(CH_3)OCH_3$ | Smp.81-82° |
| 2.54 | – | $C(CH_3)=CH\ CH_2OH$ (trans) | $N(CH_3)OCH_3$ | Oel |
| 2.55 | – | $C(CH_3)=CH_2$ | $N(CH_2)OCH_3$ | Smp.56-59° |
| 2.56 | – | $C(CH_3)=CH_2$ | $N(C_2H_5)_2$ | Smp.113° |

(Fortsetzung)

| Vbg.No. | X | $R_4$ | $NR_1R_2$ | phys. Daten |
|---|---|---|---|---|
| 2.57 | - | 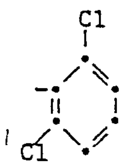 | $N(CH_3)OCH_3$ | Smp.62-65° |
| 2.58 | - | 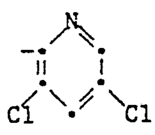 | $N(CH_3)OCH_3$ | Smp.87-90° |
| 2.59 | - | 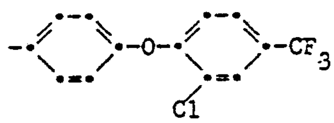 | $N(CH_3)OCH_3$ | Smp.118-120° |
| 2.60 | - | 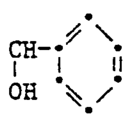 | $N(CH_3)OCH_3$ | Oel |
| 2.61 | - | $C(CH_3)_3$ | $N(CH_3)OCH_3$ | Smp. 100-2° |
| 2.62 | - | $C_4H_{9n}$ | $N(CH_3)OCH_3$ | Oel |
| 2.63 | - | 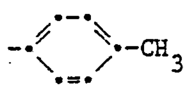 | $N(CH_3)OCH_3$ | Smp.113° |
| 2.64 | - | $C(CH_3)_2-OCH(CH_3)O\ C_2H_5$ | $N(CH_3)OCH_3$ | Oel |
| 2.65 | - | $C(CH_3)_2OH$ | $N(C_2H_5)_2$ | Smp.118° |
| 2.66 | - | CH(OH)- 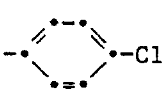 | $N(CH_3)OCH_3$ | Oel |
| 2.67 | - | $CH(OCH_3)$- 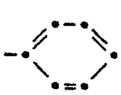 | $N(CH_3)OCH_3$ | Oel |

(Fortsetzung)

| Vbg.No. | X | $R_4$ | $NR_1R_2$ | phys. Daten |
|---|---|---|---|---|
| 2.68 | - | $C(CH_3)_2O-$ 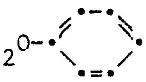 | $N(CH_3)OCH_3$ | Oel |
| 2.69 | - | $C(CH_3)_2O-$ 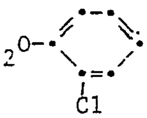 | $N(CH_3)OCH_3$ | Oel |
| 2.70 | - | $C(CH_3)_2-NH_2$ | $N(CH_3)OCH_3$ | Smp. 77-80° |
| 2.71 | - | $CH(CH_3)NHCH_3$ | $N(CH_3)OCH_3$ | Oel |
| 2.72 | - | $CH_2N(CH_3)_2$ | $N(CH_3)OCH_3$ | Smp. 61° |
| 2.73 | - | $CH(CH_3)NH-$ 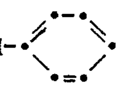 | $N(CH_3)OCH$ | Smp. 110° |
| 2.74 | - | $C(CH_3)_2NH-$ 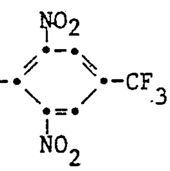 | $N(CH_3)OCH_3$ | Smp. 141° |
| 2.75 | - | $C(CH_3)_2-NHCON(CH_3)OCH_3$ | $N(CH_3)OCH_3$ | Smp. 128-132° |
| 2.76 | - | $C(C_2H_5)_2NHCON(CH_3)OCH_3$ | $N(CH_3)OCH_3$ | Smp. 89-93° |
| 2.77 | - | $C(CH_3)_2NH-COSC_2H_5$ | $N(CH_3)OCH_3$ | Smp. 102-105° |
| 2.78 | - | $C(CH_3)_2NHCOOC_2H_5$ | $N(CH_3)OCH_3$ | |
| 2.79 | - | $C(CH_3)_2NH-CONHCH_3$ | $N(CH_3)OCH_3$ | Smp. 163° |
| 2.80 | - | $C(C_2H_5)_2NHCONHCH_3$ | $N(CH_3)OCH_3$ | Smp. 110° |
| 2.81 | - | $C(CH_3)_2NHCONHC_2H_5$ | $N(CH_3)OCH_3$ | Oel |
| 2.82 | - | $C(CH_3)(OCH_3)CH_2CH(CH_3)_2$ | $N(CH_3)OCH_3$ | Oel |
| 2.83 | - | $C(C_2H_5)OH$ | $N(CH_3)OCH_3$ | Smp. 96° |
| 2.84 | - | 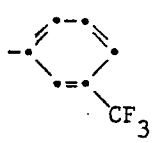 | $N(CH_3)OCH_3$ | Smp. 71° |
| 2.85 | - | 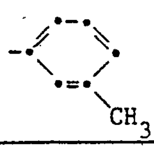 | $N(CH_3)OCH_3$ | Smp. 85° |

(Fortsetzung)

| Vbg.No. | X | $R_4$ | $NR_1R_2$ | phys. Daten |
|---|---|---|---|---|
| 2.86 | 4-Cl | 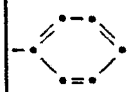 | $N(CH_3)_2$ | Smp. 161-163° |
| 2.87 | - | $C(CH_3)_2OH$ | $N(CH_3)_2$ | Smp. 115° |
| 2.88 | 4-Cl | $C(CH_3)_2OH$ | $N(CH_3)_2$ | Smp. 137° |
| 2.89 | 4-$CH_3$ | $C(CH_3)_2OH$ | $N(CH_3)_2$ | Smp. 127° |
| 2.90 | 4-$CH_3$ | $C(CH_3)_2OH$ | $NHCH_3$ | Smp. 146° |
| 2.91 | 4-Br | $C(CH_3)_2OH$ | $N(CH_3)_2$ | |
| 2.92 | - | 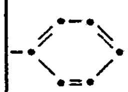 | $NHCH_3$ | Smp. 172-174° |
| 2.93 | - | $C(CH_3)(CH=CH_2)OCH(OC_2H_5)CH_3$ | $N(CH_3)OCH_3$ | Oel |
| 2.94 | - | $C(CH_3)=CH-CH_2Cl$ | $N(CH_3)OCH_3$ | Smp. 58-60° |
| 2.95 | - | $CH(CH_3)OH$ | $N(CH_3)OCH_3$ | Oel |
| 2.96 | - | 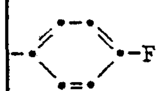 | $N(CH_3)OCH_3$ | Smp. 97° |
| 2.97 | - | 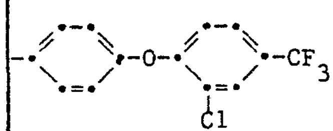 | $N(CH_3)_2$ | |
| 2.98 | - | $-C(CH_3)_2NHCONHC_4H_9t.$ | $N(CH_3)OCH_3$ | Smp. 165-166° |
| 2.99 | - | $-C(C_2H_5)_2NHCONHC_4H_9t.$ | $N(CH_3)OCH_3$ | Smp. 175-180° |
| 2.100 | - | $-C(CH_3)_2NHCONHC_3H_7n$ | $N(CH_3)OCH_3$ | Smp. 105-110° |
| 2.101 | - | $-C(C_2H_5)_2NHCONHC_3H_7n$ | $N(CH_3)OCH_3$ | Smp. 114-118° |

(Fortsetzung)

| Vbg.No. | X | $R_4$ | $NR_1R_2$ | phys. Daten |
|---|---|---|---|---|
| 2.102 | – | $C(C_2H_5)_2NHCONH$- 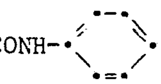 | $N(CH_3)OCH_3$ | |
| 2.103 | – | $C(C_2H_5)_2NHCONHC_2H_5$ | $N(CH_3)OCH_3$ | Smp. 124° |
| 2.104 | – | $C(CH_3)_2NHCOOCH_3$ | $N(CH_3)OCH_3$ | |
| 2.105 | – | $C(CH_3)_2NHCONH$- 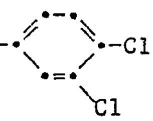 | $N(CH_3)OCH_3$ | Smp. 180-185° |
| 2.106 | – | $C(CH_3)_2NHCONH$- 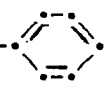 | $N(CH_3)OCH_3$ | Smp. 68-70° |
| 2.107 | – | $C(CH_3)_2NHCOOCH_3$ | $N(CH_3)_2$ | |
| 2.108 | – | $C(C_2H_5)_2NHCOOC_2H_5$ | $N(CH_3)_2$ | |
| 2.109 | – | $-C(CH_3)_2NHSO_2CH_3$ | $N(CH_2)OCH_3$ | Smp. 110-114° |
| 2.110 | – | -CH——N-CO-$NOCH_3$<br>\| \| \|<br>$CH_3$ $CH_3$ $CH_3$ | $N(CH_3)OCH_3$ | Harz |
| 2.111 | – | $C(C_2H_5)_2NHCOOC_2H_5$ | $N(CH_3)OCH_3$ | |
| 2.112 | – | $C(C_2H_5)_2NHCOOCH_3$ | $N(CH_3)_2$ | |
| 2.113 | – | $C(C_2H_5)_2NHCOOCH_3$ | $N(CH_3)OCH_3$ | |
| 2.114 | – | $C(CH_3)_2NHCOOC_4H_9iso$ | $N(CH_3)OCH_3$ | |
| 2.115 | – | $C(CH_3)_2NHCOOC_4H_9n$ | $N(CH_3)OCH_3$ | |

(Fortsetzung)

| Vbg.No. | X | $R_4$ | $NR_1R_2$ | phys. Daten |
|---|---|---|---|---|
| 2.116 | - | $C(CH_3)_2NHCOOC_4H_9$iso | $N(CH_3)_2$ | |
| 2.117 | - | $C(CH_3)_2NHCOOC_4H_9$n | $N(CH_3)_2$ | |
| 2.118 | - | $C(CH_3)_2NHCOOC_3H_7$iso | $N(CH_3)OCH_3$ | |
| 2.119 | - | $C(CH_3)_2NHCOOC_3H_7$iso | $N(CH_3)_2$ | |
| 2.120 | - | $CH(CH_3)N(CH_3)COOC_2H_5$ | $N(CH_3)OCH_3$ | |
| 2.121 | - | $CH(CH_3)N(CH_3)COOCH_3$ | $N(CH_3)OCH_3$ | |
| 2.122 | - | $CH(CH_3)N(CH_3)COOC_4H_9$n | $N(CH_3)OCH_3$ | |
| 2.123 | - | $CH(CH_3)N(CH_3)COOC_3H_7$iso | $N(CH_3)OCH_3$ | |
| 2.124 | - | $C(CH_3)_2N(CH_3)COOCH_3$ | $N(CH_3)_2$ | |
| 2.125 | - | $C(CH_3)_2N(CH_3)COOC_2H_5$ | $N(CH_3)OCH_3$ | |
| 2.126 | - | $C(CH_3)_2N(CH_3)COOC_4H_9$n | $N(CH_3)OCH_3$ | |
| 2.127 | - | $C(CH_3)_2N(CH_3)COOC_3H_7$iso | $N(CH_3)_2$ | |
| 2.128 | - | $C(CH_3)_2N(CH_3)COOCH_3$ | $N(CH_3)OCH_3$ | |
| 2.129 | - | $C(CH_3)_2N(CH_3)COOC_2H_5$ | $N(CH_3)_2$ | |
| 2.130 | - | $C(CH_3)_2N(CH_3)COOC_4H_9$n | $N(CH_3)_2$ | |
| 2.131 | - | $C(CH_3)_2N(CH_3)COOC_3H_7$iso | $N(CH_3)OCH_3$ | |

Beispiel 7

Herstellung einiger Aufbereitungsformen :

Granulat

Zur Herstellung eines 5 %igen Granulates werden die folgenden Stoffe verwendet :

5 Teile N-[3-(3'-Hydroxy-3'-methylbut-1'-in-1'-yl)-phenyl]-N'-methoxy-N'-methyl-harnstoff
0,25 Teile epoxidiertes Pflanzenöl
0,25 Teile Cetylpolyglykoläther,
3,50 Teile Polyäthylenglykol,
91 Teile Kaolin (Korngrösse : 0,3-0,8 mm).

Die Aktivsubstanz wird mit dem Pflanzenöl vermischt und in 6 Teilen Aceton gelöst, hierauf wird Polyäthylenglykol und Cetylpolyglykoläther zugesetzt. Die so erhaltene Lösung wird auf Kaolin aufgesprüht und anschliessend im Vakuum verdampft.

Spritspulver

Zur Herstellung eines a) 70 %igen und b) 10 %igen Spritzpulvers werden folgende Bestandteile verwendet :

a) 70 Teile N-[3-(3'-Methyl-but-3'-en-1'-in-1'-yl)-phenyl]-N'-methoxy-N'-methyl-harnstoff,
5 Teile Natriumdibutylnaphthylsulfonat,
3 Teile Naphthalinsulfonsäuren-Phenolsulfonsäuren-Formaldehyd-Kondensat 3 : 2 : 1,
10 Teile Kaolin,
12 Teile Champagne-Kreide ;

b) 10 Teile des obigen Wirkstoffes,
3 Teile Gemisch der Natriumsalze von gesättigten Fettalkoholsulfaten,
5 Teile Naphthalinsulfonsäuren-Formaldehyd-Kondensat,
82 Teile Kaolin.

Der angegebene Wirkstoff wird auf die entsprechenden Trägerstoffe (Kaolin und Kreide) aufgezogen und anschliessend mit den übrigen Bestandteilen vermischt und vermahlen. Man erhält Spritzpulver von vorzüglicher Benetzbarkeit und Schwebefähigkeit. Aus solchen Spritzpulvern können durch Verdünnen mit Wasser Suspensionen von 0,1-80 % Wirkstoff erhalten werden, die sich zur Unkrautbekämpfung in Pflanzenkulturen eignen.

Paste

Zur Herstellung einer 45 %igen Paste werden folgende Stoffe verwendet :

45 Teile N-[3-Chlor-4-(3'-methyl-3'-hydroxybut-1'-in-1'-yl)-phenyl]-N'-methoxy-N'-methyl-harnstoff,
5 Teile Natriumaluminiumsilikat,
14 Teile Cetylpolyglykoläther mit 8 Mol Aethylenoxid,
1 Teil Oleylpolyglykoläther mit 5 Mol Aethylenoxid,
2 Teile Spindeloel,
10 Teile Polyäthylenglykol,
23 Teile Wasser.

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Emulsionskonzentrat

Zur Herstellung eines 25 %igen Emulsionskonzentrates werden

25 Teile N-[3-(4'-methylphenyläthinyl)-phenyl]-N'-methoxy-N'-methyl-harnstoff,
5 Teile einer Mischung von Nonylphenolpolyoxyäthylen und Calciumdodecylbenzolsulfat,
15 Teile Cyclohexanon,
55 Teile Xylol.

miteinander vermischt. Dieses Konzentrat kann mit Wasser zu Emulsionen mit geeigneten Konzentrationen von z. B. 0,1 bis 10 % verdünnt werden. Solche Emulsionen eignen sich zur Bekämpfung von Unkräutern in Kulturpflanzungen.

Beispiel 8

Prüfung der herbiziden Wirkung.

Pre-emergente Herbizid-Wirkung (Keimhemmung)

Im Gewächshaus wird unmittelbar nach der Einsaat der Versuchspflanzen in Töpfe die Erdoberfläche mit einer wässerigen Dispersion des Wirkstoffes erhalten aus einem 25 %igen Emulsionskonzentrat oder aus einem 25 %igen Spritzpulver mit Wirkstoffen, die wegen ungenügender Löslichkeit nicht als Emulsionskonzentrat hergestellt werden können, gespritzt. Es werden verschiedene Konzentrationen angewendet und die Menge Wirkstoff wird in kg pro Hektar ausgerechnet. Die Töpfe werden dann im Gewächshaus bei 22-25 °C und 50-70 % relativer Luftfeuchtigkeit gehalten und regelmässig bewässert. Der Versuch wird nach 3 Wolchen ausgewertet.

Post-emergente Herbizid-Wirkung (Kontaktherbizid)

Eine grössere Anzahl Unkräuter und Kulturpflanzen, sowohl monokotyle wie dicotyle, wurden im Gewächshaus in Töpfen gezogen und nach dem Auflaufen (im 4- bis 6-Blattstadium) mit einer wässerigen Wirkstoffdispersion in verschiedenen Dosierungen, ausgedrückt in kg Wirksubstanz pro Hektare, auf die Pflanzen gespritzt und diese bei 24-26 °C und 45-60 % relativer Luftfeuchtigkeit gehalten. Der Versuch wird 2 Wochen nach der Behandlung ausgewertet.

Die Verbindungen der Formel I zeigten bei einen Aufwandmenge von 4 kg/ha starke herbizide Wirkung, sowohl im Vor- wie auch im Nachauflaufverfahren.

**Ansprüche**

1. Aethinylphenylharnstoffe der Formel I,

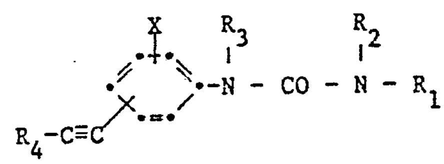

(I)

worin

X Wasserstoff, Halogen, $C_1$-$C_4$ Alkyl, $C_1$-$C_4$ Alkoxy, $C_1$-$C_4$ Haloalkyl oder Nitro,

$R_1$ Wasserstoff, $C_1$-$C_6$ Alkyl, $C_1$-$C_4$ Alkoxy, $C_2$-$C_6$ Alkenyl oder Phenyl, welches halogensubstituiert sein kann,

$R_2$ Wasserstoff, $C_1$-$C_6$ Alkyl, $C_3$-$C_6$ Cycloalkyl, $C_2$-$C_6$ Alkenyl, $C_2$-$C_6$ Alkinyl oder Benzyl, welches halogensubstituiert sein kann oder

$R_1$ und $R_2$ zusammen mit dem Stickstoffatom, an das sie gebunden sind, auch einen Pyrrolidin-, Piperidin-, Morpholin- oder Piperazinring bilden kann,

$R_3$ Wasserstoff oder $C_1$-$C_4$ Alkyl,

$R_4$ Wasserstoff, $C_1$-$C_{12}$ Alkyl welches unsubstituiert oder durch Hydroxy, Halogen, Pyranyloxy, Furanyloxy, Phenyl, Phenoxy, $C_1$-$C_4$ Alkoxy, $C_2$-$C_8$ Alkoxyalkoxy, $C_1$-$C_4$ Alkoxycarbonyl oder eine Aminogruppe $—N(R_1)R_2$, $—N(R_1)COOR_2$, $—N(R_1)COSR_2$ oder durch $—CON(R_1)R_2$ substituiert ist,

— Phenyl oder Pyridyl, welches unsubstituiert oder substituiert durch Halogen, $C_1$-$C_4$ Alkyl, $C_1$-$C_4$ Alkoxy, $C_1$-$C_4$ Haloalkyl, Nitro, $C_1$-$C_4$ Alkylsulfonyl oder Phenoxy substituiert ist, wobei Phenoxy unsubstituiert oder wie Phenyl substituiert ist,

— $C_2$-$C_{12}$ Alkenyl, welches unsubstituiert oder durch Halogen, Hydroxy, $C_1$-$C_4$ Alkoxy, $C_2$-$C_8$ Alkoxyalkyl oder $C_1$-$C_4$ Alkoxycarbonyl substituiert ist,

— $C_3$-$C_{12}$ Cycloalkyl oder Cycloalkenyl, welches mono- oder bicyclisch ist und durch Halogen oder Hydroxy oder $C_1$-$C_4$ Alkoxy substituiert sein kann.

2. Aethinylphenylharnstoffe gemäss Anspruch 1 der Formel Ia

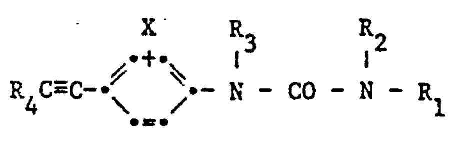

(Ia)

worin $R_1$, $R_2$, $R_3$, $R_4$ und X die unter Formel I, Anspruch 1 gegebene Bedeutung haben.

3. Aethinylphenylharnstoffe gemäss Anspruch 1 der Formel Ia

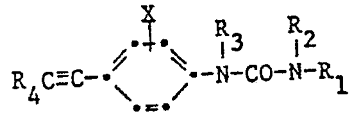

(Ia)

worin X Wasserstoff, $R_1$ Wasserstoff, $C_1$-$C_6$ Alkyl, $C_2$-$C_6$ Alkenyl oder $C_1$-$C_4$ Alkoxy, $R_2C_1$-$C_6$ Alkyl oder $C_2$-$C_6$ Alkinyl, $R_3$ Wasserstoff und $R_4$ Wasserstoff, $C_1$-$C_{12}$ Alkyl, gegebenenfalls substituiert durch Hydroxyl, Halogen, $C_1$-$C_4$ Alkoxy, $C_2$-$C_8$ Alkoxyalkoxy, Phenyl, gegebenenfalls substituiert durch Halogen, $C_1$-$C_4$ Alkyl, $C_1$-$C_4$ Alkoxy, oder $C_1$-$C_4$ Haloalkyl oder eine Gruppe —CONH—Phenyl.

4. Aethinylphenylharnstoffe gemäss Anspruch 1 der Formel Ib

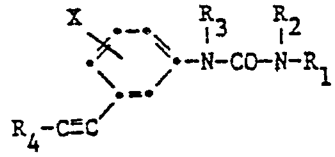

(Ib)

worin $R_1$, $R_2$, $R_3$, $R_4$ und X die unter Formel I, Anspruch 1 gegebene Bedeutung haben.

5. Aethinylphenylharnstoffe gemäss Anspruch 1 der Formel

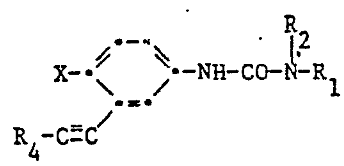

worin X Wasserstoff, Halogen oder $C_1$-$C_4$ Alkyl, $R_1$ Wasserstoff, $C_1$-$C_6$ Alkyl, $C_2$-$C_6$ Alkenyl oder $C_1$-$C_4$ Alkoxy, $R_2$ $C_1$-$C_6$ Alkyl oder $C_2$-$C_6$ Alkenyl,

$R_4$ Wasserstoff, $C_1$-$C_{12}$ Alkyl, welches unsubstituiert oder durch Hydroxyl, Halogen, $C_1$-$C_4$ Alkoxy, $C_2$-$C_8$ Alkoxyalkoxy oder durch eine Aminogruppe $—N(R_1)R_2$ substituiert ist,

— Phenyl, welches unsubstituiert oder durch Halogen, $C_1$-$C_4$ Alkyl, $C_1$-$C_4$ Alkoxy oder $C_1$-$C_4$ Haloalkyl substituiert ist;

— $C_2$-$C_{12}$ Alkenyl, welches unsubstituiert oder durch Halogen, Hydroxyl, $C_1$-$C_4$ Alkoxy, $C_2$-$C_8$ Alkoxyalkoxy oder $C_1$-$C_4$ Alkoxycarbonyl substituiert ist, oder $C_3$-$C_6$ Cycloalkyl oder Cycloalkenyl.

6. Aethinylphenylharnstoffe gemäss Anspruch 1 der Formel Ic

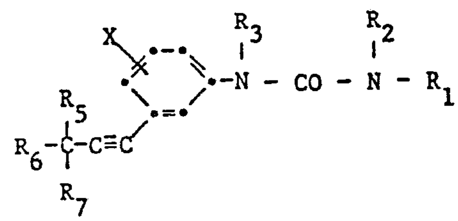

(Ic)

worin $R_1$, $R_2$, $R_3$ und X die unter Formel I, Anspruch 1 gegebene Bedeutung haben und

$R_5$ Wasserstoff, $C_1$-$C_6$ Alkyl, $C_2$-$C_6$ Alkenyl,

$R_6$ dasselbe wie $R_5$ oder Phenyl das unsubstituiert oder durch Halogen, $C_1$-$C_4$ Alkyl, $C_1$-$C_4$ Alkoxy, $C_1$-$C_4$ Haloalkyl oder Nitro oder Phenoxy substituiert ist,

$R_5$ und $R_6$ zusammen mit dem Kohlenstoffatom an das sie gebunden sind, auch einen $C_3$-$C_{12}$ Cycloalkyl- oder Cycloalkenylring darstellen kann, der mono- oder bicyclisch ist und der durch Halogen, Hydroxy oder $C_1$-$C_4$ Alkoxy substituiert sein kann,

$R_7$ Hydroxyl, $C_1$-$C_6$ Alkyl welches durch Halogen oder Hydroxy substituiert und durch Sauerstoff ein- oder mehrfach unterbrochen sein kann, wobei die Anzahl Kohlenstoffatome in den Resten $R_5$, $R_6$ und $R_7$ 11 nicht übersteigt.

7. Aethinyl-phenylharnstoffe gemäss Anspruch 1 der Formel Id

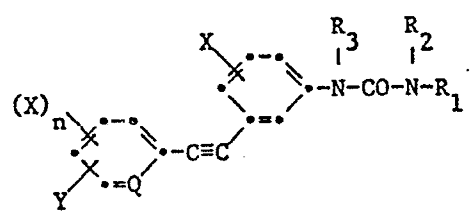

(Id)

worin $R_1$, $R_2$, $R_3$ und X die unter Formel I, Anspruch 1 gegebene Bedeutung haben, n 1 oder 2 ist und Y Wasserstoff, Halogen, Nitro, oder einen gegebenenfalls durch Halogen, Nitro, $C_1$-$C_4$ Alkyl, $C_1$-$C_4$ Alkoxy, oder $C_1$-$C_4$ Haloalkyl ein- oder mehrfach substituierten Phenoxyrest, und Q die Methingruppe oder das Stickstoffatom bedeutet.

8. Verfahren zur Herstellung der Aethinylphenylharnstoffe der Formel I, Anspruch 1, in denen $R_3$ Wasserstoff bedeutet, dadurch gekennzeichnet, dass man ein Aethinylphenylderivat der Formel II

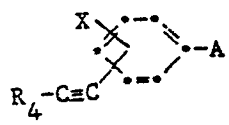

(II)

mit einem Amin der Formel III

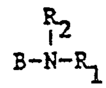

(III)

in an sich bekannter Weise umsetzt, wobei $R_1$, $R_2$, $R_4$ und X die unter Formel I, Anspruch 1 gegebene Bedeutung haben, während A und B Reste sind, die unter Anlagerung oder Kondensation eine Harnstoffgruppe bilden.

9. Verfahren zur Herstellung der Aethinyl-phenylharnstoffe der Formel I, Anspruch 1, dadurch gekennzeichnet, dass man einen halogenierten Phenylharnstoff der Formel IV

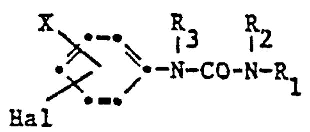

(IV)

worin $R_1$, $R_2$, $R_3$ und X die unter Formel I, Anspruch 1 gegebene Bedeutung haben, und Hal ein sich in meta- oder para-Position zur Harnstoffgruppe befindliches Halogenatom bedeutet, in Gegenwart einer Base und eines Katalysators mit einer Aethinylverbindung der Formel V

$$R_4—C{\equiv}CH \qquad (V)$$

umsetzt, worin $R_4$ die unter Formel I, Anspruch 1, gegebene Bedeutung hat.

10. Verfahren zur Herstellung der Aethinyl-phenylharnstoffe der Formel I, Anspruch 1, dadurch gekennzeichnet, dass man einen Phenylharnstoff der Formel IV

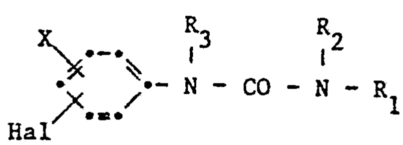

(IV)

worin $R_1$, $R_2$, $R_3$ und X die unter Formel I, Anspruch 1 gegebene Bedeutung haben, in Gegenwart einer starken, anorganischen Base und gegebenenfalls in Gegenwart eines Katalysators in einem inerten Lösungsmittel mit einem tertiären Aethinylalkanol der Formel VI unter Abspaltung der Ketogruppe $O = CR'_5R'_6$ umsetzt,

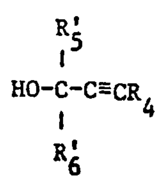

(VI)

wobei $R'_5$ $C_1$-$C_6$ Alkyl oder $C_2$-$C_6$ Alkenyl,

$R'_6$ dasselbe wie $R'_5$ oder Phenyl, das unsubstituiert oder durch Halogen, $C_1$-$C_4$ Alkyl, $C_1$-$C_4$ Alkoxy, $C_1$-$C_4$ Haloalkyl, Nitro oder Phenoxy substituiert ist, $R_5$ und $R_6$ zusammen mit dem Kohlenstoffatom, an das sie gebunden sind auch einen $C_3$-$C_{12}$ Cycloalkyl oder Cycloalkenylrest darstellen kann, der mono- oder bicyclisch ist und der durch Halogen, Hydroxy oder $C_1$-$C_4$ Alkoxy substituiert sein kann und $R_4$ die in Anspruch 1 gegebene Bedeutung hat.

11. Verfahren zur Herstellung von Aethinylharnstoffen der Formel I, Anspruch 1, dadurch gekennzeichnet, dass man eine Verbindung der Formel VII

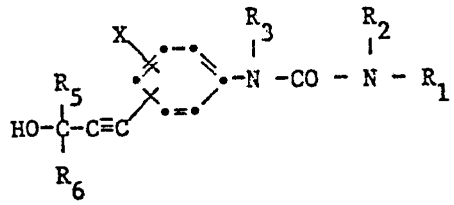

(VII)

worin $R_1$, $R_2$, $R_3$, $R_5$, $R_6$ und X die in Ansprüchen 1 und 6 gegebene Bedeutung haben, mit einer starken anorganischen Base und gegebenenfalls in Gegenwart eines Katalysators, in einem inerten Lösungsmittel bis zur Abspaltung der Gruppe $O = CR_5R_6$ reagieren lässt und gegebenenfalls den erhaltenen Aethinyl-phenylharnstoff der Formel VIII

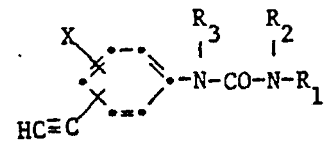

(VIII)

worin $R_1$, $R_2$, $R_3$ und X die unter Formel I, Anspruch 1 gegebene Bedeutung haben, in Gegenwart einer Base und eines Katalysators mit einem halogenierten Rest Hal-$R'_4$, worin Hal ein Halogenatom bedeutet und $R'_4$ die unter Formel I, Anspruch 1 für $R_4$ mit Ausnahme von Wasserstoff gegebene Bedeutung hat, umsetzt.

12. Herbizides Mittel, dadurch gekennzeichnet, dass es als Wirkstoff mindestens einen Aethinyl-phenylharnstoff der Formel I, Anspruch 1 enthält.

13. Die Verwendung der Aethinyl-phenylharnstoffe der Formel I, Anspruch 1, zur Bekämpfung unerwünschten Pflanzenwachstums.

14. Die Verwendung gemäss Anspruch 13 der Aethinyl-phenylharnstoffe der Formel I, Anspruch 1 zu selektiven Bekämpfung von Unkräutern in Kulturen von Nutzpflanzen.

15. Die Verwendung der Aethinyl-phenylharnstoffe der Formel I, Anspruch 1 zur Dessikation und Defoliation von Baumwoll- und Kartoffelpflanzen, vor deren Ernte.

16. Die Verwendung gemäss Anspruch 14 der Aethinyl-phenylharnstoffe der Formel I, Anspruch 1 zur selektiven Bekämpfung von Unkräutern in Kulturen von Baumwolle und Zuckerrüben.

17. Die Verwendung gemäss Anspruch 14 der Aethinyl-phenylharnstoffe der Formel I, Anspruch 1 zur selektiven Bekämpfung von Unkräutern in Kulturen von Getreide.

**Claims**

1. Ethynylphenylureas of the formula I

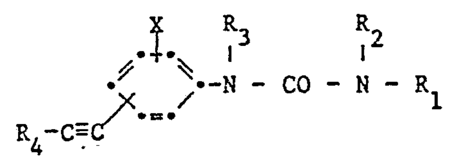

(I)

wherein

X is hydrogen, halogen, $C_1$-$C_4$-alkyl, $C_1$-$C_4$-alkoxy, $C_1$-$C_4$-haloalkyl or nitro ;

$R_1$ is hydrogen, $C_1$-$C_6$-alkyl, $C_1$-$C_4$-alkoxy, $C_2$-$C_6$-alkenyl, or phenyl unsubstituted or substituted by halogen ;

$R_2$ is hydrogen, $C_1$-$C_6$-alkyl, $C_3$-$C_6$-cycloalkyl, $C_2$-$C_6$-alkenyl or $C_2$-$C_6$-alkynyl, or benzyl unsubstituted or substituted by halogen ; or

$R_1$ and $R_2$ together with the nitrogen atom to which they are bound can also form a pyrrolidine, piperidine, morpholine or piperazine ring ;

$R_3$ is hydrogen or $C_1$-$C_4$-alkyl :

$R_4$ is hydrogen, $C_1$-$C_{12}$-alkyl unsubstituted or substituted by hydroxyl, halogen, pyranyloxy, furanyloxy, phenyl, phenoxy, $C_1$-$C_4$-alkoxy, $C_2$-$C_8$-alkoxyalkoxy, $C_1$-$C_4$-alkoxycarbonyl or an amino group $—N(R_1)R_2$, $—N(R_1)COOR_2$, $—N(R_1)COSR_2$ or $—CON(R_1)R_2$, or

$R_4$ is phenyl or pyridyl each unsubstituted or substituted by halogen, $C_1$-$C_4$-alkyl, $C_1$-$C_4$-alkoxy, $C_1$-$C_4$-haloalkyl, nitro, $C_1$-$C_4$-alkylsulfonyl or phenoxy which is unsubstituted or substituted like phenyl or

$R_4$ is $C_2$-$C_{12}$-alkenyl, unsubstituted or substituted by halogen, hydroxyl, $C_1$-$C_4$-alkoxy, $C_2$-$C_8$-alkoxyalkyl or $C_1$-$C_4$-alkoxycarbonyl, or

$R_4$ is $C_3$-$C_{12}$-cycloalkyl or $C_3$-$C_{12}$-cycloalkenyl each of which can be mono- or bicyclic and can be substituted by halogen, hydroxyl or $C_1$-$C_4$-alkoxy.

2. Ethynylphenylureas according to Claim 1 of the formula Ia

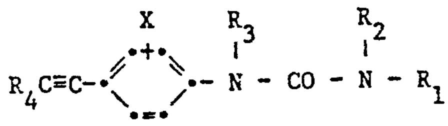 (Ia)

wherein $R_1$, $R_2$, $R_3$, $R_4$ and X have the meanings defined under the formula I, Claim 1.

3. Ethynylphenylureas according to Claim 1 of the formula Ia

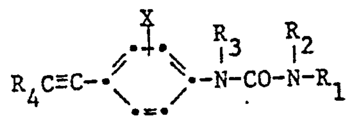 (Ia)

wherein X is hydrogen, $R_1$ is hydrogen, $C_1$-$C_6$-alkyl, $C_2$-$C_6$-alkenyl or $C_1$-$C_4$-alkoxy, $R_2$ is $C_1$-$C_6$-alkyl or $C_2$-$C_6$-alkynyl, $R_3$ is hydrogen, and $R_4$ is hydrogen, $C_1$-$C_{12}$-alkyl unsubstituted or substituted by hydroxyl, halogen, $C_1$-$C_4$-alkoxy or $C_2$-$C_8$-alkoxyalkoxy, or $R_4$ is phenyl unsubstituted or substituted by halogen, $C_1$-$C_4$-alkyl, $C_1$-$C_4$-alkoxy or $C_1$-$C_4$-haloalkyl or a group —CONH—phenyl.

4. Ethynylphenylureas according to Claim 1 of the formula Ib

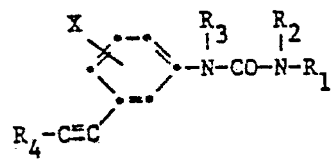 (Ib)

wherein $R_1$, $R_2$, $R_3$, $R_4$ and X have the meanings defined under the formula I, Claim 1.

5. Ethynylphenylureas according to Claim 1 of the formula

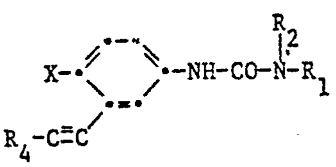

wherein X is hydrogen, halogen or $C_1$-$C_4$-alkyl, $R_1$ is hydrogen, $C_1$-$C_6$-alkyl, $C_2$-$C_6$-alkenyl or $C_1$-$C_4$-alkoxy, $R_2$ is $C_1$-$C_6$-alkyl or $C_2$-$C_6$-alkenyl, and

$R_4$ is hydrogen, $C_1$-$C_{12}$-alkyl unsubstituted or substituted by hydroxyl, halogen, $C_1$-$C_4$-alkoxy or $C_2$-$C_8$-alkoxyalkoxy or by an amino group —$N(R_1)R_2$, or

$R_4$ is phenyl unsubstituted or substituted by halogen, $C_1$-$C_4$-alkyl, $C_1$-$C_4$-alkoxy or $C_1$-$C_4$-haloalkyl, or

$R_4$ is $C_2$-$C_{12}$-alkenyl unsubstituted or substituted by halogen, hydroxyl, $C_1$-$C_4$-alkoxy, $C_2$-$C_8$-alkoxyalkoxy or $C_1$-$C_4$-alkoxycarbonyl, or $R_4$ is $C_3$-$C_{12}$-cycloalkyl or $C_3$-$C_{12}$-cycloalkenyl.

6. Ethynylphenylureas according to Claim 1 of the formula Ic

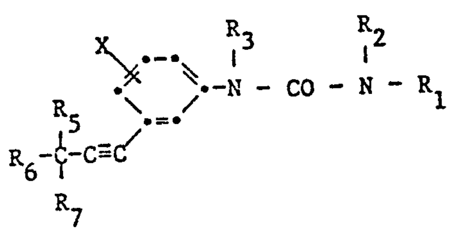 (Ic)

wherein $R_1$, $R_2$, $R_3$ and X have the meanings defined under the formula I, Claim 1, and

$R_5$ is hydrogen, $C_1$-$C_6$-alkyl or $C_1$-$C_6$-alkenyl,

$R_6$ is the same as $R_5$, or it is phenyl unsubstituted or substituted by halogen, $C_1$-$C_4$-alkyl, $C_1$-$C_4$-alkoxy, $C_1$-$C_4$-haloalkyl, nitro or phenoxy, or

$R_5$ and $R_6$ together with the carbon atom to which they are bound can also form a $C_3$-$C_{12}$-cyckoalkyl or $C_3$-$C_{12}$-cycloalkenyl ring each of which can be mono- or bicyclic and can be substituted by halogen, hydroxyl or $C_1$-$C_4$-alkoxy,

$R_7$ is hydroxyl or $C_1$-$C_6$-alkyl which can be substituted by halogen or hydroxyl and once or repeatedly interrupted by oxygen, the number of carbon atoms in the radicals $R_5$, $R_6$ and $R_7$ not exceeding 11.

7. Ethynylphenylureas according to Claim 1 of the formula Id

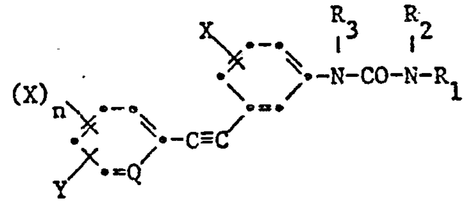

(Id)

wherein $R_1$, $R_2$, $R_3$ and X have the meanings defined under the formula I, Claim 1, n is 1 or 2, and Y is hydrogen, halogen or nitro, or a phenoxy group unsubstituted or mono- or polysubstituted by halogen, nitro, $C_1$-$C_4$-alkyl, $C_1$-$C_4$-alkoxy, or $C_1$-$C_4$-haloalkyl, and Q is the methine group or the nitrogen atom.

8. Process for producing the ethynylphenylureas of the formula I, Claim 1, in which $R_3$ is hydrogen, by reaction of an ethynylphenyl derivative of the formula II

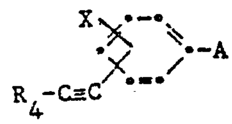

(II)

with an amine of the formula III

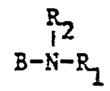

(III)

in a manner known per se, wherein $R_1$, $R_2$, $R_4$ and X have the meanings defined under the formula I, Claim 1, and A and B are radicals which form a urea group as a result of addition or condensation.

9. Process for producing the ethynylphenylureas of the formula I, Claim 1, wherein a halogenated phenylurea of the formula IV

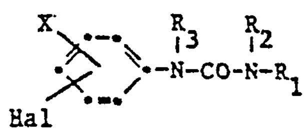

(IV)

wherein $R_1$, $R_2$, $R_3$ and X have the meanings defined under the formula I, Claim 1, and « Hal » is a halogen atom which is in the meta- or para-position with respect to the urea group, is reacted, in the presence of a base and of a catalyst, with an ethynyl compound of the formula V

$$R_4—C{\equiv}CH \qquad (V)$$

wherein $R_4$ has the meaning defined under the formula I, Claim 1.

10. Process for producing the ethynylphenylureas of the formula I, Claim 1, wherein a phenylurea of the formula IV

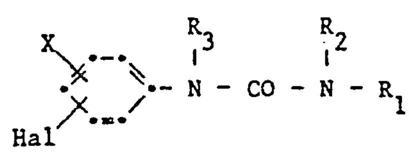

(IV)

wherein $R_1$, $R_2$, $R_3$ and X have the meanings given under the formula I, Claim 1, is reacted, in the presence of a strong inorganic base and optionally in the presence of a catalyst in an inert solvent, with a tertiary ethynyl alkanol of the formula VI, with the splitting-off of the keto group $O = CR'_5R'_6$,

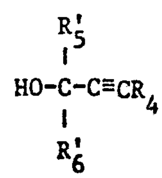

(VI)

wherein $R'_5$ is $C_1$-$C_6$-alkyl or $C_2$-$C_6$-alkenyl,

$R'_6$ is the same as $R'_5$ or phenyl which is unsubstituted or substituted by halogen, $C_1$-$C_4$-alkyl, $C_1$-

$C_4$-alkoxy, $C_1$-$C_4$-haloalkyl, nitro or phenoxy, $R_5$ and $R_6$ together with the carbon atom to which they are bound can also form a $C_3$-$C_{12}$-cycloalkyl or $C_3$-$C_{12}$-cycloalkenyl group each of which can be mono- or bicyclic and can be substituted by halogen, hydroxyl or $C_1$-$C_4$-alkoxy, and

$R_4$ has the meaning given in Claim 1.

11. Process for producing ethynylphenylureas of the formula I, Claim 1, wherein a compound of the formula VII

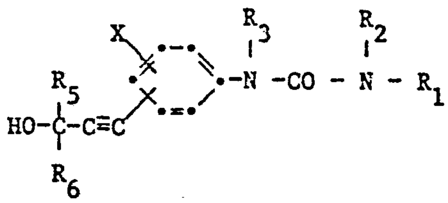

(VII)

wherein $R_1$, $R_2$, $R_3$, $R_5$, $R_6$ and X have the meanings defined in Claims 1 and 6, is reacted with a strong inorganic base and optionally in the presence of a catalyst, in an inert solvent, until the keto group $O = CR_5R_6$ is split off ; and optionally the resulting ethynylphenylurea of the formula VIII

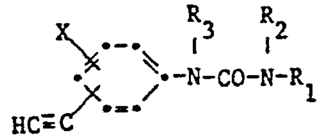

(VIII)

wherein $R_1$, $R_2$, $R_3$ and X have the meanings defined under the formula I, Claim 1, is reacted, in the presence of a base and of a catalyst, with a halogenated radical Hal-$R'_4$, wherein « Hal » is a halogen atom, and $R'_4$ has the meaning given under the formula I, Claim 1 for $R_4$, with the exception of hydrogen.

12. Herbicidal composition containing as active ingredient at least one ethynylphenylurea of the formula I, Claim 1.

13. The use of the ethynylphenylureas of the formula I, Claim 1, for combating undesirable plant growth.

14. The use according to Claim 13 of the ethynylphenylureas of the formula I, Claim 1, for selectively combating weeds in crops of cultivated plants.

15. The use of the ethynylphenylureas of the formula I, Claim 1, for dessication and defoliation of cotton and potato plants before harvesting.

16. The use according to Claim 14 of the ethynylphenylureas of the formula I, Claim 1, for selectively combating weeds in cotton crops and sugar beet crops.

17. The use according to Claim 14 of the ethynylphenylureas of the formula I, Claim 1, for selectively combating weeds in cereal crops.

**Revendications**

1. Ethynyl-phénylurées de formule I

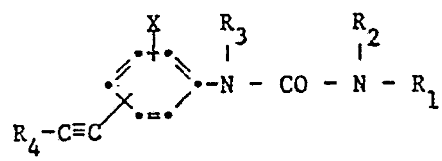

(I)

dans laquelle

X représente l'hydrogène, un halogène, un groupe alkyle en $C_1$-$C_4$, alcoxy en $C_1$-$C_4$, halogénoalkyle en $C_1$-$C_4$ ou nitro,

$R_1$ représente l'hydrogène, un groupe alkyle en $C_1$-$C_6$, alcoxy en $C_1$-$C_6$, alcényle en $C_2$-$C_6$ ou phényle, qui peut être substitué par des halogènes,

$R_2$ représente l'hydrogène, un groupe alkyle en $C_1$-$C_6$, cycloalkyle en $C_3$-$C_6$, alcényle en $C_2$-$C_6$, alcynyle en $C_2$-$C_6$ ou benzyle, qui peut être substitué par des halogènes, ou bien

$R_1$ et $R_2$ peuvent également former ensemble et avec l'atome d'azote sur lequel ils sont fixés un cycle pyrrolidine, pipéridine, morpholine ou pipérazine,

$R_3$ représente l'hydrogène ou un groupe alkyle en $C_1$-$C_4$,

$R_4$ représente l'hydrogène, un groupe alkyle en $C_1$-$C_{12}$, non substitué ou substitué par des groupes hydroxy, des atomes d'halogènes, des groupes pyrannyloxy, furannyloxy, phényle, phénoxy, alcoxy en $C_1$-$C_4$, alcoxyalcoxy en $C_2$-$C_8$, alcoxycarbonyle en $C_1$-$C_4$ ou un groupe amino —$N(R_1)R_2$, —$N(R_1)COOR_2$, —$N(R_1)COSR_2$ ou par un groupe —$CON(R_1)R_2$,

— un groupe phényle ou pyridyle, non substitué ou substitué par des halogènes, des groupes alkyle en $C_1$-$C_4$, alcoxy en $C_1$-$C_4$, halogénoalkyle en $C_1$-$C_4$, nitro, alkylsulfonyle en $C_1$-$C_4$ ou phénoxy, le groupe phénoxy pouvant être non substitué ou substitué comme le groupe phényle ;

— un groupe alcényle en $C_2$-$C_{12}$ non substitué ou substitué par des halogènes, des groupes hydroxy, alcoxy en $C_1$-$C_4$, alcoxyalkyle en $C_2$-$C_8$ ou alcoxycarbonyle en $C_1$-$C_4$.

— un groupe cycloalkyle ou cycloalcényle en $C_3$-$C_{12}$, mono- ou bi-cyclique, et qui peut être substitué par des halogènes ou des groupes hydroxy ou alcoxy en $C_1$-$C_4$.

2. Ethynyl-phénylurées selon la revendication 1, de formule Ia

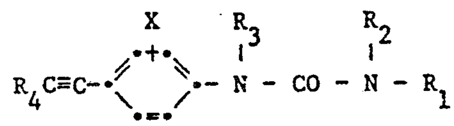

(Ia)

dans laquelle $R_1$, $R_2$, $R_3$, $R_4$ et X ont les significations indiquées en référence à la formule I dans la revendication 1.

3. Ethynyl-phénylurées selon la revendication 1, de formule Ia

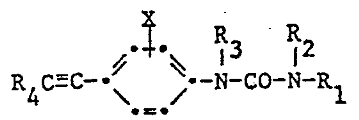

(Ia)

dans laquelle X représente l'hydrogène, $R_1$ représente l'hydrogène, un groupe alkyle en $C_1$-$C_6$, alcényle en $C_2$-$C_6$ ou alcoxy en $C_1$-$C_4$, $R_2$ représente un groupe alkyle en $C_1$-$C_6$ ou alcynyle en $C_2$-$C_6$, $R_3$ représente l'hydrogène et $R_4$ l'hydrogène, un groupe alkyle en $C_1$-$C_{12}$ éventuellement substitué par des groupes hydroxy, des halogènes, des groupes alcoxy en $C_1$-$C_4$, alcoxyalcoxy en $C_2$-$C_8$, un groupe phényle éventuellement substitué par des halogènes, des groupes alkyle en $C_1$-$C_4$, alcoxy en $C_1$-$C_4$ ou halogénoalkyle en $C_1$-$C_4$, ou un groupe —CONH—phényle.

4. Ethynyl-phénylurées selon la revendication 1, de formule Ib

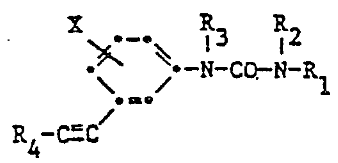

(Ib)

dans laquelle $R_1$, $R_2$, $R_3$, $R_4$ et X ont les significations indiquées en référence à la formule I dans la revendication 1.

5. Ethynyl-phénylurées selon la revendication 1, de formule

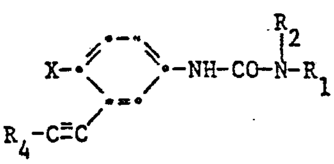

dans laquelle X représente l'hydrogène, un halogène ou un groupe alkyle en $C_1$-$C_4$, $R_1$ représente l'hydrogène, un groupe alkyle en $C_1$-$C_6$, alcényle en $C_2$-$C_6$ ou alcoxy en $C_1$-$C_4$, $R_2$ représente un groupe alkyle en $C_1$-$C_6$ ou alcényle en $C_2$-$C_6$,

$R_4$ représente l'hydrogène, un groupe alkyle en $C_1$-$C_{12}$ non substitué ou substitué par des groupes hydroxy, des atomes d'halogènes, des groupes alcoxy en $C_1$-$C_4$, alcoxyalcoxy en $C_2$-$C_8$ ou par un groupe amino-$N(R_1)R_2$,

— un groupe phényle non substitué ou substitué par des halogènes, des groupes alkyle en $C_1$-$C_4$, alcoxy en $C_1$-$C_4$ ou halogénoalkyle en $C_1$-$C_4$,

— un groupe alcényle en $C_2$-$C_{12}$ non substitué ou substitué par des halogènes, des groupes hydroxy, alcoxy en $C_1$-$C_4$, alcoxyalcoxy en $C_2$-$C_8$ ou alcoxycarbonyle en $C_1$-$C_4$, ou un groupe cycloalkyle ou cycloalcényle en $C_3$-$C_6$.

6. Ethynyl-phénylurées selon la revendication 1, de formule Ic

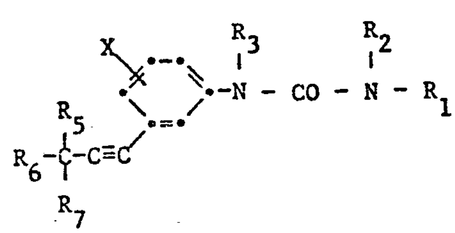

(Ic)

dans laquelle $R_1$, $R_2$, $R_3$ et X ont les significations indiquées en référence à la formule I dans la revendication 1, et

$R_5$ représente l'hydrogène, un groupe alkyle en $C_1$-$C_6$, alcényle en $C_2$-$C_6$.

$R_6$ a les mêmes significations que $R_5$ ou représente un groupe phényle non substitué ou substitué par des halogènes, des groupes alkyle en $C_1$-$C_4$, alcoxy en $C_1$-$C_4$, halogénoalkyle en $C_1$-$C_4$ ou nitro ou phénoxy,

$R_5$ et $R_6$ peuvent également représenter ensemble et avec l'atome de carbone sur lequel ils sont fixés un cycle cycloalkyle ou cycloalcényle en $C_3$-$C_{12}$, mono- ou bi-cyclique et qui peut être substitué par des halogènes, des groupes hydroxy ou alcoxy en $C_1$-$C_4$.

$R_7$ représente un groupe hydroxy, alkyle en $C_1$-$C_6$ substitué par des halogènes ou des groupes hydroxy ou interrompu une ou plusieurs fois par l'oxygène, le nombre des atomes de carbone des restes $R_5$, $R_6$ et $R_7$ ne dépassant pas 11.

7. Ethynyl-phénylurées selon la revendication 1, de formule Id

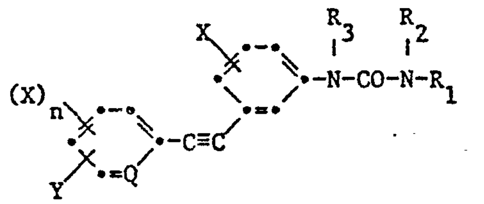

(Id)

dans laquelle $R_1$, $R_2$, $R_3$ et X ont les significations indiquées en référence à la formule I dans la revendication 1, n est égal à 1 ou 2 et Y représente l'hydrogène, un halogène, un groupe nitro ou un reste phénoxy éventuellement mono- ou poly-substitué par des halogènes, des groupes nitro, alkyle en $C_1$-$C_4$, alcoxy en $C_1$-$C_4$ ou halogénoalkyle en $C_1$-$C_4$ et Q représente le groupe méthine ou l'atome d'azote.

8. Procédé de préparation des éthynyl-phénylurées de formule I, revendication 1, dans laquelle $R_3$ représente l'hydrogène, caractérisé en ce que l'on fait réagir de manière connue en soi un dérivé éthynyl-phénylique de formule II

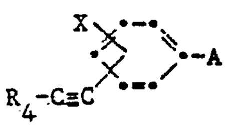

(II)

avec une amine de formule III

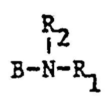

(III)

$R_1$, $R_2$, $R_4$ et X ayant les significations indiquées en référence à la formule I dans la revendication 1 et A et B étant des restes qui forment un groupe urée par addition ou par condensation.

9. Procédé de préparation des éthynyl-phénylurées de formule I, revendication 1, caractérisé en ce que l'on fait réagir une phénylurée halogénée de formule IV

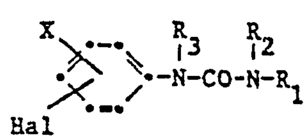

(IV)

dans laquelle $R_1$, $R_2$, $R_3$ et X ont les significations indiquées en référence à la formule I dans la revendication 1 et Hal représente un atome d'halogène en position méta ou para du groupe urée, en présence d'une base et d'un catalyseur, avec un composé éthynylique de formule V

$$R_4{-}C \equiv CH \qquad (V)$$

dans laquelle $R_4$ a les significations indiquées en référence à la formule I dans la revendication 1.

10. Procédé de préparation des éthynyl-phénylurées de formule I, revendication 1, caractérisé en ce qu'on fait réagir une phénylurée de formule IV

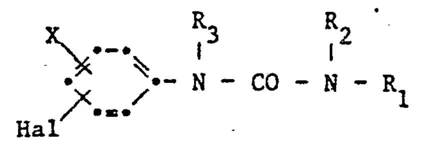

(IV)

dans laquelle $R_1$, $R_2$, $R_3$ et X ont les significations indiquées en référence à la formule I dans la revendication 1, en présence d'une base minérale forte et éventuellement en présence d'un catalyseur, dans un solvant inerte, avec un alcanol éthynylique tertiaire de formule VI, avec séparation du groupe céto $O = CR'_5R'_6$,

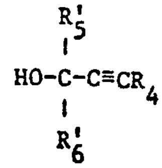

(VI)

dans laquelle $R'_5$ représente un groupe alkyle en $C_1$-$C_6$ ou alcényle en $C_2$-$C_6$,

$R'_6$ a les mêmes significations que $R'_5$ ou représente un groupe phényle non substitué ou substitué par des halogènes, des groupes alkyle en $C_1$-$C_4$, alcoxy en $C_1$-$C_4$, halogénoalkyle en $C_1$-$C_4$, nitro ou phénoxy, $R_5$ et $R_6$ pouvant également représenter ensemble et avec l'atome de carbone sur lequel ils sont fixés un reste cycloalkyle ou cycloalcényle en $C_3$-$C_{12}$, mono- ou bi-cyclique et qui peut être substitué par des halogènes, des groupes hydroxy ou alcoxy en $C_1$-$C_4$, et

$R_4$ a les significations indiquées dans la revendication 1.

11. Procédé de préparation des éthynyl-phénylurées de formule I, revendication 1, caractérisé en ce que l'on fait réagir un composé de formule VII

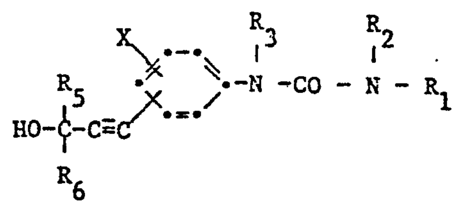

(VII)

dans laquelle $R_1$, $R_2$, $R_3$, $R_5$, $R_6$ et X ont les significations indiquées dans les rev. 1 et 6, avec une base minérale forte et éventuellement en présence d'un catalyseur, dans un solvant inerte, jusqu'à élimination du groupe $O = CR_5R_6$, et on fait réagir éventuellement l'éthynyl-phénylurée obtenue, de formule VIII

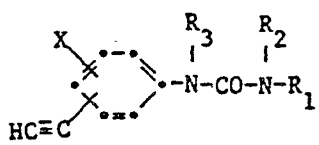

(VIII)

dans laquelle $R_1$, $R_2$, $R_3$ et X ont les significations indiquées en référence à la formule I dans la revendication 1, en présence d'une base et d'un catalyseur, avec un reste halogéné Hal-$R'_4$, dans lequel Hal représente un atome d'halogène et $R'_4$ a les significations indiquées pour $R_4$ en référence à la formule I dans la revendication 1, à l'exception de l'hydrogène.

12. Produit herbicide caractérisé en ce qu'il contient en tant que substance active au moins une éthynyl-phénylurée de formule I, revendication 1.

13. L'utilisation des éthynyl-phénylurées de formule I, revendication 1, dans la lutte contre la croissance de végétaux indésirables.

14. L'utilisation selon la revendication 13 des éthynyl-phénylurées de formule I, revendication 1, dans la lutte sélective contre les mauvaises herbes dans les cultures de végétaux utiles.

15. L'utilisation des éthynyl-phénylurées de formule I, revendication 1, pour la dessication et la défoliation des plants de coton et de pommes de terre avant la récolte.

16. L'utilisation selon la revendication 14, des éthynyl-phénylurées de formule I, revendication 1, pour la lutte sélective contre les mauvaises herbes dans les cultures de coton et de betteraves à sucre.

17. L'utilisation selon la revendication 14, des éthynyl-phénylurées de formule I, revendication 1, pour la lutte sélective contre les mauvaises herbes dans les cultures de céréales.